Europäisches Patentamt

European Patent Office

Office européen des brevets

(19)

(11) **EP 1 312 681 A1**

(12) **EUROPEAN PATENT APPLICATION**

(43) Date of publication:
**21.05.2003 Bulletin 2003/21**

(51) Int Cl.$^7$: **C12Q 1/68**

(21) Application number: **01127195.4**

(22) Date of filing: **16.11.2001**

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **Boehringer Ingelheim International GmbH**
**55216 Ingelheim (DE)**

(72) Inventors:
• **Dickson, Barry**
**1040 Wien (AT)**

• **Berger, Juerg**
**1070 Wien (AT)**
• **Suzuki, Takashi**
**1030 Wien (AT)**
• **Knoblich, Juergen**
**1030 Wien (AT)**

(74) Representative: **Laudien, Dieter, Dr. et al**
**Boehringer Ingelheim GmbH,**
**CD Patents**
**55216 Ingelheim am Rhein (DE)**

(54) **Method for identifying therapeutic targets by use of genetic screens in Drosophila melanogaster**

(57)    A method for obtaining disease-related mammalian target gene is based on prior identification of a Drosophila melanogaster gene that is involved in a molecular process or pathway related to the disease. Mapping of the Drosophila gene is achieved by a genetic cross between Drosophila strains that contain one or more P element insertions that allow recombination events within a defined genomic interval. The set of polymorphisms identified in the recombinant progeny allows for rapid, systematic, and high-resolution genetic mapping in Drosophila.

Fig. 1

EP 1 312 681 A1

## Description

### Field of the invention

The invention relates to methods for genetic mapping in the model organism Drosophila melanogaster, as a means to identify novel conserved genes useful as therapeutic targets in the treatment of human diseases.

More specifically, the invention relates to the use of certain Drosophila strains, sequence polymorphisms, and SNP genotyping assays that facilitate the identification of novel Drosophila genes. The methods can be used to identify genes in Drosophila that are implicated in physiological processes of relevance to human diseases. Identification of these Drosophila genes allows the subsequent identification of their human orthologues, which may then be used to develop therapeutic agents for the treatment of these human diseases.

### Background of the invention

There is a need for systematic methods to identify drug targets by exploiting the specific genetic alterations that characterize disease-relevant molecular mechanisms. To this end, several genetic screening methods in model organisms have been developed. Large-scale, systematic genetic screens in model organisms provide a means for functionally analyzing genes and gene products within an organism that relate to a physiological process of interest. This approach has been used routinely to dissect physiologically important pathways in a number of species including the fruit fly *Drosophila melanogaster.*

Due to the evolutionary conservation of major biochemical pathways, Drosophila melanogaster has been proven to be a useful model system for the identification and characterisation of novel genes and molecular processes of relevance for human health.

As discussed in WO 01/51604, for example, genetic screens in *Drosophila* have led to the discovery of novel components of cancer associated signal transduction pathways, including the *ras* (Karim *et al*., 1996), *Notch* (Go and Artavanis-Tsakonas,1998), *dpp* (Raftery *et al*., *1995),* and *hedgehog* (Hooper and Scott, 1989) pathways. The physiological relevance of *Drosophila* tumors to those of animals has been demonstrated by studies of the *lats* tumor suppressor gene. Mutations in the *lats* gene in *Drosophila* were first discovered in genetic screens (Xu and Rubin, 1993). Human and mouse homologues of the *Drosophila lats* gene were identified and the physiological relevance of tumor suppressor genes in *Drosophila* with counterparts in mammals was confirmed.

In a recent survey of 289 human genes that are mutated, altered, amplified or deleted in a variety of human diseases, 177 (61%) were found to have orthologues (i.e. functionally-related homologues) in Drosophila melanogaster (Rubin et al., 2000b). Drosophila thus offers the opportunity to study the physiological, cellular and molecular functions of these and other disease genes in greater detail than is possible in humans, human cells, or mammalian models. More importantly, genetic mutagenesis experiments in Drosophila can be used to identify other genes that act in the same biochemical pathway. These Drosophila genes can then be used to identify their human orthologues. Although these may not be mutated in any human disorder, they are nevertheless likely to contribute to disease pathology. These genes and their products may therefore be useful targets for therapeutic agents to be used in the treatment of these diseases.

Usually, a genetic screen comprises the following steps: once a suitable Drosophila model for the human disease has been established by one of the approaches outlined below, it can then be used to identify novel Drosophila genes that contribute to this condition. Mutations in these genes can be recovered using a variety of different chemical mutagenesis strategies (Greenspan, 1997); a common strategy is to search for loss-of-function mutations that dominantly modify the phenotype that results from misexpression of a human disease gene or its Drosophila orthologue. Such screens have been performed, for example, to isolate mutations that suppress phenotypes resulting from Ras activation (Karim et al., 1996) or the expression of mutant forms of human neurodegenerative disease genes (Fernandez-Funez et al., 2000). These mutagenesis screens would define genes that, when their function is impaired genetically, reduce the severity of the disease condition in the Drosophila model. Disrupting the function of the corresponding human proteins by pharmacological agents might similarly ameliorate the human disease condition.

Typically, genetic screens in Drosophila melanogaster comprise, as a first step, a model system in which animals are genetically altered and selected according to disease-related phenotype. In the next step, mutations are recovered that modify that phenotype.

From this recovery step, a collection of chemically-induced mutations are obtained that modify or mimic the human disease condition in Drosophila. These mutations disrupt the functions of unknown Drosophila genes. Before the human or or other mammalian homologues can be identified - and consequently pharmacological agents to treat the relevant diseases can be developed - it is first necessary to identify the mutated Drosophila genes. This step - the genetic mapping and "positional cloning" (i.e. positive identification of the mutated gene) of Drosophila genes defined only by their mutant phenotype - is presently one of the major bottlenecks in this approach. This is due to the time-consuming, error-prone and ad hoc nature of traditional methods for genetic mapping in Drosophila, and the difficulties in aligning the genetic and cytological maps with the genome sequence.

## Summary of the invention

It has been an object of the invention to overcome the shortcomings of the methods available for genetic mapping and "positional cloning" in Drosophila.

To solve the problems underlying the invention, the presence of sequence polymorphisms (single nucleotide polymophisms, or SNPs, and insertions/deletions, InDels) that have been identified in the Drosophila genome have been exploited to provide a method for rapid, systematic, and high-resolution genetic mapping in Drosophila that can be readily integrated into methods for identifying disease-related human genes.

Thus, the present invention relates to a method for producing a human cDNA molecule derived from a gene that is causally involved in a human disease by

A. identifying a Drosophila melanogaster gene that is involved in a molecular process or pathway related to a disease of interest by

1. obtaining genetically altered flies that exhibit a disease-related phenotype by

a) expressing in flies a human gene known to be implicated in the disease, or its Drosophila homologue, is in its normal or mutated form such that expression of the gene results in a gene-specific phenotype, or

b) randomly mutating flies and identifying flies that exhibit a disease-related phenotype,

2. mutagenizing animals obtained in 1.a) and recovering mutations that modify said phenotype of 1.a); or recovering animals that result in phenotype 1.b),

3. mapping the mutated Drosophila gene, and

4. cloning the Drosophila gene;

B. identifying the orthologue human gene and cloning and amplifying the human cDNA.

Step A.3 comprises the steps of

(a) setting up a genetic cross between the Drosophila strain containing the mutation recovered in step 2., and another strain that is sufficiently divergent at the DNA sequence level, and that contains one or more P element insertions that allow recombination events within a defined genomic interval to be identified;

(b) recovering a series of recombinant progeny from this cross, each of which contains a genome that is a mixture of the two parental strains,

(c) identifying a set of sequence polymorphisms that discriminates between the two parental strains;

(d) determining the phenotype of some or all of the recombinant progeny

(e) assaying for some or all of the sequence polymorphisms identified in (c) in some or all of the recombinant progeny.

## Detailed description of the invention

The embodiments of step A. 1.a) and b) represent two methodologies of Drosophila melanogaster assays, the selection of which depends upon the available prior knowledge of genes and molecular pathways involved in the human disease of interest.

The embodiment of step A.1.a) represents the scenario when one or more genes have been implicated in a given disease. In this case, it is possible to establish a Drosophila model for this disease based on the misexpression of the human disease gene in Drosophila, or of its Drosophila homologue, in either their normal or mutated forms. For example, the Drosophila genome encodes homologues of the Ras oncogene (Neuman-Silberberg et al., 1984), the p53 tumor suppressor gene (Brodsky et al., 2000; Jin et al., 2000; Ollmann et al., 2000). Expression of activated forms of Drosophila Ras leads to excessive cell proliferation, as in human cancers (Karim and Rubin, 1998). Similarly, interfering with Drosophila p53 function blocks apoptosis in response to DNA damage, analogous to the failure of the cell cycle checkpoint in response to DNA damage in human cells lacking p53 (Brodsky et al., 2000; Ollmann et al., 2000). The Drosophila genome also encodes homologues of genes known mutated in neurodegenerative disorders such as Alzheimers, Huntingtons and Parkinsons diseases (Adams et al., 2000; Chan and Bonini, 2000). The functions of these genes in the fly are unknown, but expression of mutated forms of the human genes also results in neurodegeneration in Drosophila (Jackson et al., 1998; Warrick et al., 1998; Feany and Bender, 2000; Wittmann et al., 2001). These examples clearly illustrate the manner in which normal or mutant human or Drosophila genes can be used to establish Drosophila models for human diseases.

The embodiment of step A.1.b) is used when there is no prior knowledge of the genes involved in the human disease of interest. In this case, it may nevertheless be possible to establish a Drosophila model based on the cellular defects underlying the disease. For example, it is possible to identify in Drosophila defects in cellular proliferation (Xu et al., 1995; Moberg et al., 2001; Tapon et al., 2001) or neurodegeneration (Min and Benzer, 1999).

In step 2.), once such models are established, mutations can be sought that affect these phenotypes. These mutations can be induced at random by chemical or radiation treatment according to known methods, e. g. as described in WO01/51604. In the embodiment of steop A.1.a, these mutations can be screened for modification of the "disease" phenotype in Drosophila (step A.2.). This approach has been described in WO 01/51604; genetic alteration of the animal according to step A.1.a) may may be carried out according to known methods, e.g. as described in WO 01/51604 for obtaining "recombinant animals" or recombinantly modified animals", as termed therein. In the embodiment of step A.1.b), mutations can be directly screened for the disease-related phenotype. These screens may take the form of standard homozygous loss of function screens, for example, (Min and Benzer, 1999), or employ more sophisticated genetic methods to generate mosaic animals in which only small patches of cells are homozygous mutant (Chou et al., 1993; Xu and Rubin, 1993). In this latter approach, homozygous mutant clones may be generated in a random manner (Xu et al., 1995), or specifically in the tissue of interest (Newsome et al., 2000; Tapon et al., 2001).

In step 3.) these newly-induced random mutations are mapped, and in step 4.) the mutated gene is identified.

According to step A.3.a), a strain is considered sufficiently divergent from the strain carrying the mutation if it contains on average at least one DNA sequence polymorphism every 10 kb, which can be routinely determined by DNA sequence analysis.

A P element is a naturally occurring Drosophila transposon that has been genetically modified to carry various genetic markers (Greenspan, 1997).

For the purposes of the present invention, either one P element insertion known to lie within a genetic distance of approximately 5cM from the mutation of interest, or two P element insertions that flank the mutation at any distance, may be used. P elements carrying dominant genetic or molecular markers may be used, e. g. white+ (e.g. EP (Rorth, 1996), yellow+, or FRT (Xu and Rubin, 1993) markers. Each of these P elements may be placed either in cis or in trans to the mutation. In configurations in which two flanking P element insertions are used, each of these insertions is located either within a genetic distance of approximately 5cM from the mutation of interest, or near the proximal or distal end of the same chromosome arm.

In a preferred embodiment of the invention, which was used in the experiments, distal EP (Rorth, 1996), and proximal FRT (Xu and Rubin, 1993) insertions are used as flanking P elements. Alternatively, the proximal FRT insertions may also be used with similar EP insertions. For example, one can select from a series of EP elements across the chromosome arm, and in this way identify an element that lies distal to but near the mutation of interest. Such a EP element can be identied from a series of EP elements by phenotypic analysis of recombinants between the mutated strain and the EP strain.

Another variation of this strategy is to use two P element insertions that flank the mutation of interest, not necessarily the FRT and EP elements selected in the experiments. It is also possible to use just a single P element insertion, as illustrated in the experiments. This method is not restricted to FRT and EP elements. It is essential only that a dominant mutation at a defined sequence location is used. P elements carrying dominant markers such as white+ (as the EP elements), rosy+, yellow+, or green fluorescence protein (GFP) markers are an attractive and abundant source of such markers (Spradling et al., 1995; Spradling et al., 1999). Other types of transposable element insertions, such as Hobo elements (Smith et al., 1993), can also be used.

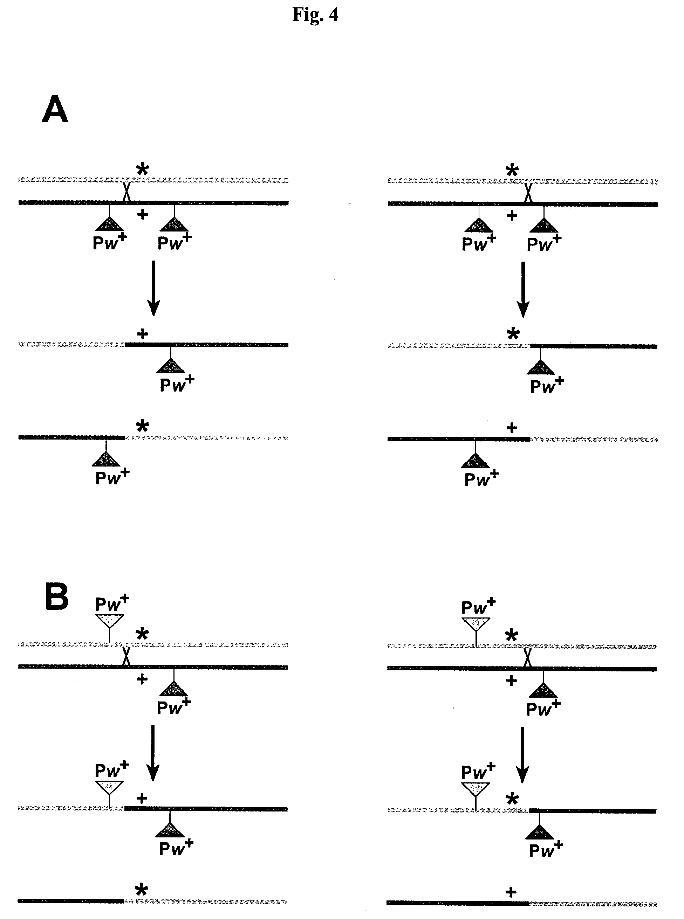

In an embodiment, a set of strains with tandem EP insertions is used for the generation of recombinants within a defined interval of 1-4 Mb; this strategy is depicted in Fig. 4: Two flanking transposon insertions (for example P{white+} elements, as shown), can be used to select for recombination events specifically within the vicinity of the mutation of interest. These two transposons may initially be placed either in cis (A) or in trans (B). In either case, 4 distinct classes of recombinant chromosomes can be derived, as illustrated. These recombinants can be identified based on the segregation of the dominant markers carried on the transposon insertions.

In a variation of this embodiment, similarly constructed strains consisting of tandem dominantly-marked transposon insertions separated by less than 4 Mb are used in order to isolate recombination events within that interval.

Alternatively, paired EP insertions in the trans configuration (i.e. one EP located on the same chromosome as the mutation, the other on the opposite chromosome) may be used.

In step A.3.b), recombinant progeny are identified by selection for recombination events either between the mutation of interest and the P element, or between the two flanking P elements. In the experiments of the invention, an eye-specific source of FLP recombinase (Newsome et al., 2000) was used to identify recombination events between a proximal FRT insertion and a distal EP insertion. This method can be used with any other pair of FRT and EP insertions. Recombination events between other pairs of P elements can be identified by virtue of the dominant markers they carry (e.g. by the different eye colours of animals carrying one or two white+-marked P elements). Recombination events between the mutation of interest and a P element can be identified by selecting on the basis of a phenotype associated with the mutation, such as lethality, or the phenotype used in step 2.), and the dominant marker carried on the P element.

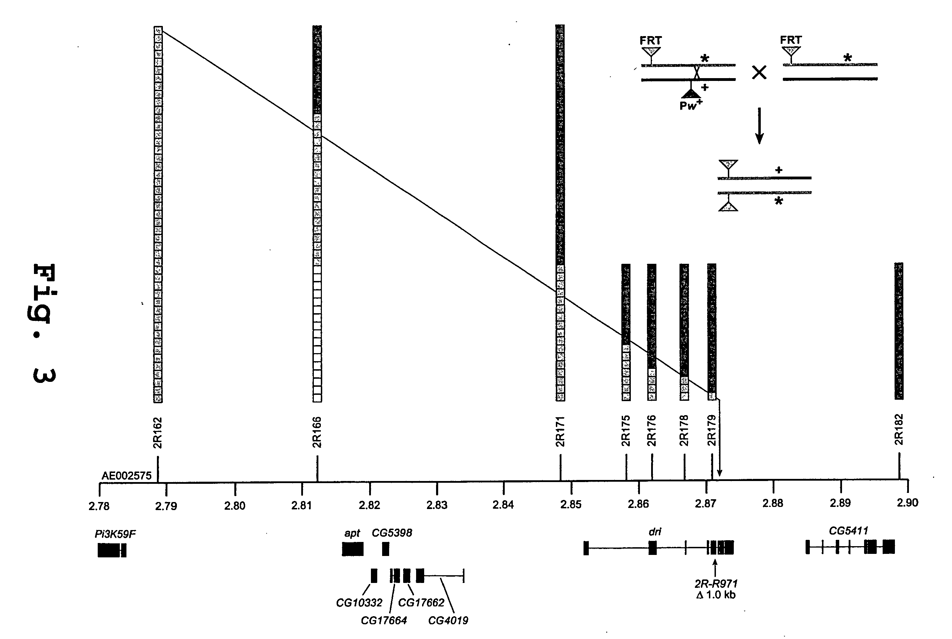

In an embodiment, the phenotype of step A.1. is associated with lethality. In this case, in step A.3.d recombinant progeny are recovered by selecting against the mutation-associated lethality. This embodiment is illustrated in the Example and in Fig. 3. In particular, the lethal phenotype may be associated to the mutation by the presence of a lethal insertion transposon within a 2 kb interval from the mutation; e.g. the transposon contains a *dead-ringer (dri)* gene insertion. In step A.3.c), sequence polymorphisms are identified by DNA sequence analysis of selected genomic regions from each of the two strains.

In the present experiments, sequence polymorphisms were identified between FRT and EP strains (Tables A - D). Tables A - D list identified polymorphisms between FRT and EP strains, and the Release 2 Drosophila genome sequence (Adams et al., 2000). The nucleotide positions of the polymorphic site in the indicated Release 2 contig are shown. Specific allelic variants present in the FRT and EP strains, and the Release 2 sequence, are listed where known. Idenitified polymorphic restriction sites are also indicated. Table A lists polymorphisms on chromosome arm 2L, Table B lists polymorphisms on 2R, Table C polymorphisms on 3L, and Table D polymorphisms on 3R.

Additional polymorphisms between these strains, or polymorphisms between other strains can be identified in the same manner. This involves the directed PCR amplification and sequencing of selected genomic fragments. PCR products of appox. 1 kb in size are sequenced in two separate reactions, each using one of the PCR primers as a sequencing primer. Individual sequence traces are called with PHRED 0.000925.c (Ewing et al., 1998a and 1998b). Pair-wise alignments to the genome sequence are prepared for POLYBAYES using CrossMatch (Marth et al., 1999). Sequences are then multiply realigned using the POLYBAYES 3.0 (release 2001-03-27) anchored alignment algorithm (Marth et al., 1999). Genome sequences are assigned a PHRED score of 40. Polymorphisms reported by POLYBAYES are retained only if the PHRED quality score of each variant nucleotide exceeded 20, and the $P_{SNP}$ value assigned by POLYBAYES exceeded 0.5 (corresponding to a PHRED score of 33 in a pair-wise alignment). FRT and EP sequence traces can also be aligned by the SeqMan program (DNAStar), or any other commercially available sequence analysis software, and visually inspected. These alignments can be used to make minor adjustments to the POLYBAYES alignment, and are particularly useful for the detection of most InDels larger than 8 nucleotides.

In step A.3.d) the presence or absence of the mutation of interest is determined according to phenotypic criteria, as in step 2.

In step A.3.e), the specific allelic variant of one or more sequence polymorphisms is determined for one or more recombinant progeny. The number of sequence polymorphisms tested will depend on the desired resolution <and the number of recombinant progeny available. Typically, between 10 and 100 sites would be assayed. The recombinant progeny can be individually or en masse. The allelic variant at each polymorphic site can be determined by any suitable method, for example by DNA sequence analysis, by PCR product length polymorphisms (PLP), or restriction fragment length polymorphisms (RFLP) as outlined below.

A PLP assay is defined as a PCR reaction that allows the two alleles to be distinguished based on the size of the resulting fragments when separated by agarose or polyacrylamide gel electrophoresis. Typically, PCR primers are designed to amplify a product of 100-300 bp, including the polymorphic InDel. The exact sizes of the PCR product amplified from each allele using these primers will therefore differ by the size of the InDel.

An RFLP assay is defined as a restriction digest of a PCR product using any commercially available restriction enzyme that results in fragments of different sizes from each of the two alleles. Typically, PCR primers are designed to amplify a product of 100-1000bp, including the polymorphic site. The precise position of these primers is selected so that the fragments generated after the restriction digest will differ for the two alleles, taking into account the number and distribution of non-polymorphic sites for the selected restriction enzyme in the region of the polymorphic restriction site.

Other currently available methods that are useful for determining polymorphisms in the present invention, such as fluoresence polarization and denaturing high performance liquid chromatography, are reviewed in (Kwok, 2001).

Table E lists verified PLP assays. For each indicated polymorphism (as listed in Tables A-D) the indicated primer pair will amplify a product of different sizes from the FRT and EP strains. The sizes of these PCR products are indicated.

Table F lists verified RFLP assays. For each indicated polymorphism (as listed in Tables A-D) PCR amplification with the indicated primer followed by restriction digest with the indicated enzyme will result in a different pattern of restriction fragments for the FRT and EP alleles.

PLP and RFLP assays for other polymorphisms can be established similarly.

In the experiments the invention, 7223 single nucleotide polymorphisms (SNPs) and 1392 insertions/deletions (InDels) in FRT and EP strains of *Drosophila* were identified. These sequence polymorphisms define a map of 787 autosomal marker loci with a resolution of 114 kb. PCR product length polymorphism (PLP) or restriction fragment length polymorphism (RFLP) assays for 215 of these markers were established. The use of this map is demonstrated by delimiting two mutations to intervals of 169 kb and 307 kb respectively.

Using a local high-density SNP map, a third mutation was also mapped to a resolution of approximately 2 kb, sufficient to localise the mutation within a single gene.

In the following, the development of the genetic mapping according to step A.3. of the invention is described in detail, i.e. the prerequisites and considerations that it was based on, the strategies, the methods and the results:

A dense map of sequence polymorphisms is a valuable resource for mapping human disease loci (Collins et al., 1997), and experimentally induced mutations in model organisms Winzeler, E.A. *et al.*, 1998; Cho, R.J. *et al.*; 1999; Wicks et al., 2001; Hoskins *et al.*,2001;Lindblad-Toh *et al.*,2000).

For genetic mapping in model organisms, SNPs and InDels offer several key advantages over conventional genetic markers: they are highly abundant, exist in only two co-dominant variants, and, in most cases, are phenotypically neutral. To enable genetic mapping with SNP markers, it is first necessary to establish a map of sequence polymorphisms between the strain used for mutagenesis and a divergent strain that can be used to generate recombinants for mapping. If laboratory strains are largely isogenic, as is the case for example in *Caenorhabditis elegans*, then a sufficiently divergent natural isolate must be selected for mapping purposes. A polymorphism map can then be constructed simply by performing light shotgun sequencing of this strain and aligning these sequences to the genome sequence of the laboratory strain (Cho et al, 1999). The difficulty in this case is that the genetic background of the new isolate is poorly defined, and does not include any visible genetic markers to assist in the recovery of recombinants (Cho et al, 1999). Ideally, well-defined laboratory strains would themselves have sufficient nucleotide diversity for mapping purposes, as shown here to be the case for *Drosophila.* However, this poses the challenge of constructing a map of polymorphisms between two strains, neither of which may correspond to the reference genome. Two general approaches to constructing such a map might be considered. One strategy would be to perform light shotgun sequencing of one strain, align this to the reference genome, and then genotype the second strain for any polymorphisms thus identified. An alternative approach is to directly amplify and resequence specific loci from each of the two strains of interest. A mathematical analysis of this problem (see the Example) indicates that the directed approach is more likely to be preferable if polymorphisms are abundant; the shotgun approach if they are rare.

In *Drosophila,* polymorphisms are sufficiently abundant to favor the directed approach. Indeed, sequence tagged sites generated during the *Drosophila* genome project (Kimmerly *et al.*, 1996). have recently been resequenced from several different wild type strains and natural isolates in order to construct a map of 474 SNP marker loci (Hoskins *et al.*,2001;Teeter *et al.*,2000). The inventors intended to construct a similar map for useful laboratory strains, also using the directed approach. One additional advantage of this strategy is that it allows different strains to be selected for different genomic regions. The inventors therefore selected a set of paired strains for each chromosome arm: one strain carrying an FRT insertion (Xu and Rubin, 1993) at the base of the arm, and a corresponding strain carrying an insertion of an EP element (Rørth. et al., 1998) at the tip. The choice of these strains was based on the following criteria: First, the FRT strains, by virtue of their utility in the generation of genetic mosaics, are frequently used as parental strains in large scale mutagenesis screens (e.g. Xu et al., 1995; Liu and Montell, 1999; Newsome et al., 2000; Benlali et al., 2000; Pichaud and Desplan, 2001). Second, traditional methods are particularly inadequate for mapping mutations that can be detected only in genetic mosaics, for which few visible markers are available and transheterozygous complementation tests are impossible. Third, the EP elements carry a dominant *white*[+] marker and, unlike many other transposon insertions, they have not been preselected for any mutant phenotype (Rørth et al., 1998). Finally, flies carrying both a proximal FRT and a distal EP insertion on the same chromosome arm can readily be identified. This greatly facilitates the recovery of a set of recombination events spanning almost an entire chromosome arm. Paired FRT and EP strains were selected for each of the four major autosomal arms. The X chromosome was omitted from this analysis, as it is less frequently included in mutagenesis screens involving genetic mosaics. Recombination is exceedingly rare on the small fourth chromosome, and so it too was excluded.

The inventors amplified and sequenced PCR products, typically ~1 kb in size, from each pair of FRT and EP homozygotes. Primers were designed to amplify non-repetitive, non-coding regions spaced at regular intervals across each autosomal arm. Sequences from the two strains were then screened for polymorphisms using POLYBAYES (Marth et al., 1999) and limited visual inspection. In total, 821 pairs of PCR products were screened. 787 (95.9%) of these contained at least one sequence polymorphism, and thus define a set of marker loci useful for genetic mapping experiments. These marker loci are evenly distributed across each of the four major autosomal arms, with an average resolution of 1 marker every 114 kb. The largest gap is 578 kb. A total of 8615 polymorphisms were identified (Tables A-D): 7223 SNPs (83.8%) and 1392 InDels (16.2%).

Sequence polymorphisms between FRT and EP strains were detected on average once every 200 bp, and between each of these strains and the Release 2 genome sequence once every 157 and 153 bp respectively. The density of polymorphisms is not uniform across each arm, being generally lower nearer the centromere and, to a lesser extent, towards the telomere. Polymorphism frequency thus correlates with the recombination rate (Begun, D.J. & Aquadro, 1992), consistent with the prediction that selective sweeps should eliminate polymorphisms more effectively in regions with lower recombination rates (Begun and Aquadro, 1992). Nevertheless, sequence polymorphisms could still be detected even in regions of low recombination. The largest gap between polymorphisms identified in any two strains was 1.86 Mb; the second largest just 804 kb. Within any 2 Mb window, the average polymorphism frequency between any pair of strains is at least one polymorphism per kb. In existing laboratory strains of *Drosophila*, sequence polymorphisms are thus sufficiently abundant and widespread to support high resolution genome-wide genetic mapping.

To facilitate mapping using the sequence polymorphisms the inventors have identified, it was next sought to establish a set of assays for rapidly genotyping a subset of those polymorphisms that discriminate between the FRT and EP strains. First, PCR product length polymorphism (PLP) assays were established for 110 of the 134 InDels of 7 or more base pairs (Table E). These PLP assays can be used to genotype 93 markers. Second, the inventors examined all SNPs for restriction site polymorphisms. This identified 1592 predicted RFLP-SNPs (46.6% of all FRT versus EP SNPs). Since most marker loci contain several SNPs, nearly all markers (83.0%) can potentially be scored using a restriction digest of PCR products. The inventors have verified 239 of these RFLP-SNPs by a restriction digest of PCR products from FRT and EP homozygotes and FRT/EP heterozygotes (Table F). These verified RFLP assays can be used to genotype 162 markers. Using either of these established PCR assays, genotypes can be determined for 215 markers on the FRT versus EP map, at an average resolution of 419 kb.

The SNP map and PCR assays established in the present invention for the FRT and EP strains will be of immediate use for mapping the many mutations that have been induced on FRT chromosomes in screens involving genetic mosaics. If mutations induced on other genetic backgrounds are to be mapped using these markers and assays, a sufficient number of these markers must also be polymorphic in other pairs of strains. To assess this, the PLP assays were used to genotype 107 InDels in several additional strains: two "wild type" strains, two strains carrying multiple recessive markers, and one "balancer" strain for each autosome. 82 (76.6%) of these InDels were indeed polymorphic between at least one other pair of strains in addition to the FRT and EP strains. Most of these markers and assays are therefore also useful for mapping mutations using different pairs of strains.

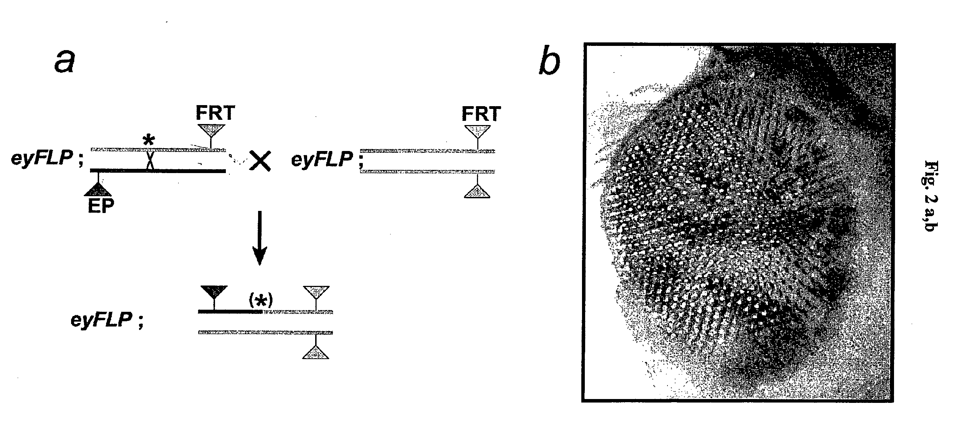

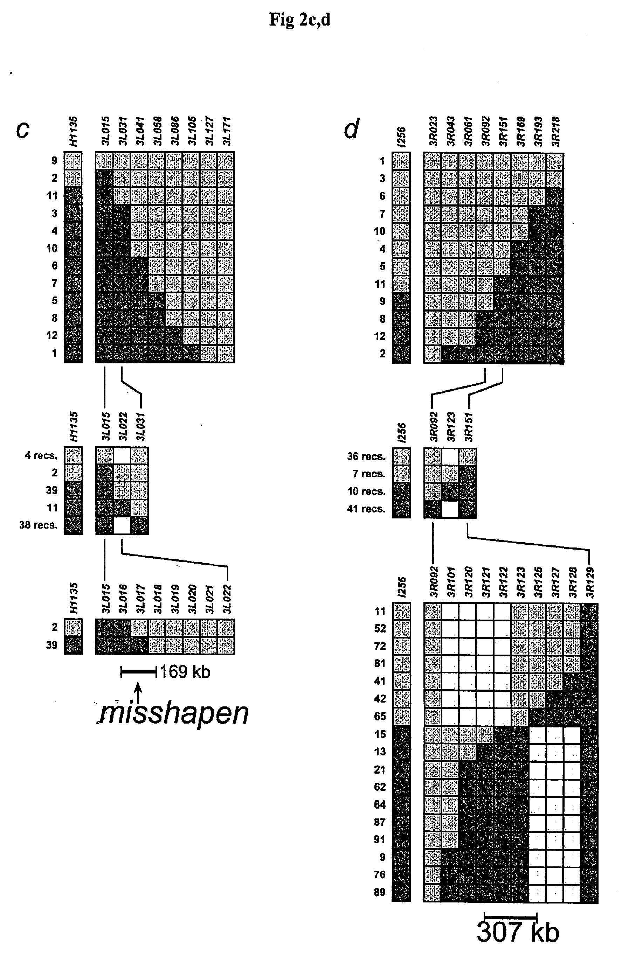

To assess the feasibility of mapping using these markers and assays, it was next sought to localize two mutations, *3L-H1135* and *3R-1256*, that had been isolated in a mosaic screen for abnormal patterns of neuronal connectivity in the adult visual system (Newsome et al., 2000). These two mutations had been induced by chemical mutagenesis on the FRT80A and FRT82B chromosomes respectively. The inventors first isolated sets of 45 and 94 recombinants, respectively, between the proximal FRT and distal EP insertions. For this, the inventors used an eye-specific source of FLP recombinase (Newsome et al., 2000) so that recombinants could be identified by their mosaicism for the *white*+ eye-colour marker located on the EP element (Fig. 2a,b). The inventors then scored each recombinant for the connectivity phenotype, and determined marker genotypes by PLP or RFLP assays, or by direct sequencing of PCR products. This analysis placed the *3L-H1135* and *3R-I256* mutations within 169 kb and 307 kb intervals, respectively (Fig. 2c,d). The region containing *3L-H1135* also includes the gene *misshapen,* which had previously been shown to function in photoreceptor axon pathfinding (Ruan et al., 1999). Complementation and phenotypic analysis identified the *3L-H1135* mutation as a new allele of *misshapen*, confirming the accuracy of this mapping strategy.

Fig. 2 depicts this experiment as follows:

*a*, Schematic of the crossing strategy used to isolate a set of recombinants spanning an entire chromosome arm. The asterisk indicates a mutation initially present on the parental FRT chromosome, and on all recombinant chromosomes for which the crossover has occurred distal to the mutation.

*b,* In the presence *of eyFLP,* eye-colour mosaicism can be used to identify recombinant chromosomes carrying both the FRT and EP insertions.

*c* and *d,* Mapping of the *3L-H1135* (*c*) and *3R-I256* (*d*) mutations. Mapping proceeded in three stages. First, a low resolution map position was obtained by the analysis of a random selection of 12 recombinants (top panels). The left columns indicate the recombinant number, and the presence (light grey) or absence (dark grey) of the visual system connectivity defect. The columns on the right show the results of PLP assays used to genotype each of the indicated SNP markers (light grey, FRT; dark grey, EP). This initial stage placed each mutation between a pair of markers separated by 1-3 Mb. All remaining recombinants were then screened for informative crossover events that had occurred between these two markers (middle panels). One intervening marker was also scored for each mutation (white, not determined). These informative recombinants were then used to obtain a high resolution map position (lower panels). In this final stage, SNP genotypes were determined by DNA sequencing.

This mapping procedure proved to be extremely rapid. Once the recombinant progeny had been generated and scored for the connectivity defect, it took only 2-3 days to map each mutation. The rate-limiting step in this procedure was the histological analysis of neuronal connectivity in each of the recombinant progeny. Clearly, more visible phenotypes could be mapped with even greater efficiency. In such cases it may also be desirable to perform PCR genotyping assays on DNA samples prepared from multiple rather than individual recombinants. While this approach sacrifices the ability to localize a mutation unambiguously between a specific pair of markers, it would allow an approximate map location to be determined within just a few hours of obtaining the recombinants.

With an average density of 1 sequence polymorphism every 150-200 bp, it will be possible to map mutations down to the sub-genic level. To assess the feasibility of such high-resolution genetic mapping, the inventors examined a third mutation from the same connectivity screen, *2R-R971.* Both the mosaic visual system connectivity defect and homozygous lethality associated with this mutation had previously been localised by traditional methods to the cytological interval 59EF. The inventors first constructed a high-resolution SNP map within this interval, comparing again the parental FRT and the distal EP chromosomes. To localise the *2R-R971* mutation on this map, the inventors generated a set of 71 recombination events between the *2R-R971* mutation and a P{*white*+} insertion within the 59D-F region (Fig. 3). Of 11 markers tested, 8 were found to discriminate between the parental *2R-R971* and P{*white*+} chromosomes. The inventors then scored these markers on the recombinant progeny, thereby placing the *2R-R971* mutation within a 2 kb interval that includes the 3' region of the *dead-ringer* (*dri*) gene (Fig. 3). Complementation, phenotypic and sequence analysis confirmed that *2R-R971* is indeed an allele *of dri.* The practical limit of SNP mapping is thus likely to be imposed only by the density of identified SNPs, rather than a lack of available polymorphisms or the presence of recombination hot-spots.

Fig. 3 shows this strategy as follows:

The inset in the upper right illustrates the strategy used to recover a set of recombinants between the *2R-R971* mutation (asterisk) and a closely linked P{*white*+} insertion. Light grey indicates the FRT chromosome carrying the *2R-R971* mutation. Dark grey indicates the P{*white*+} chromosome that carries a wild type allele at this locus (+). Black indicates the CyO balancer chromosome. The map shows nucleotide positions in the AE002575 contig, in Mb. Proximal is to the left. Boxes indicate SNP genotypes determined for each recombinant (rows) at the indicated SNP marker (columns). All SNP genotypes shown here were determined by sequence analysis (light grey, FRT; dark grey, P{*white*+}; white, not determined). Of 71 recombination events, 24 occurred proximal to the *2R162* marker (not shown), 46 occurred in the 83 kb interval between *2R162* and *2R179*, and one distal to *2R179* . The *2R-R971* mutation therefore lies distal to *2R1*79, but most likely less than 2 kb from it (given the average of one recombination event every 1.8 kb). Sequencing the entire *dri* gene from the *2R-R971* chromosome revealed a 1005 bp deletion, 661 bp distal to the *2R179* marker. No evidence of recombination hotspots could be detected at this resolution.

In summary, step A.3. of the invention comprises the use of three principal components for genetic mapping in Drosophila:

(1) a set of Drosophila strains containing dominantly marked P-element insertions that can be used to generate recombinant chromosomes for any desired genomic region - either an entire chromosome arm or just a few Mb;

(2) a set of defined sequence polymorphisms that discriminate between these strains; and (3) a set of assays that can be used to score these polymorphisms on the recombinant chromosomes.

Knowledge of the specific allele present for the mutated gene (i.e. wild type or mutant) and at defined polymorphic sites, for each member of a set of recombinant chromosomes generated using these strains, allows the mutated gene to be localized to a defined genomic interval. Provided a sufficient number of recombinant chromosomes and polymorphic sites are examined, this interval may contain only a single gene, or part of a gene.

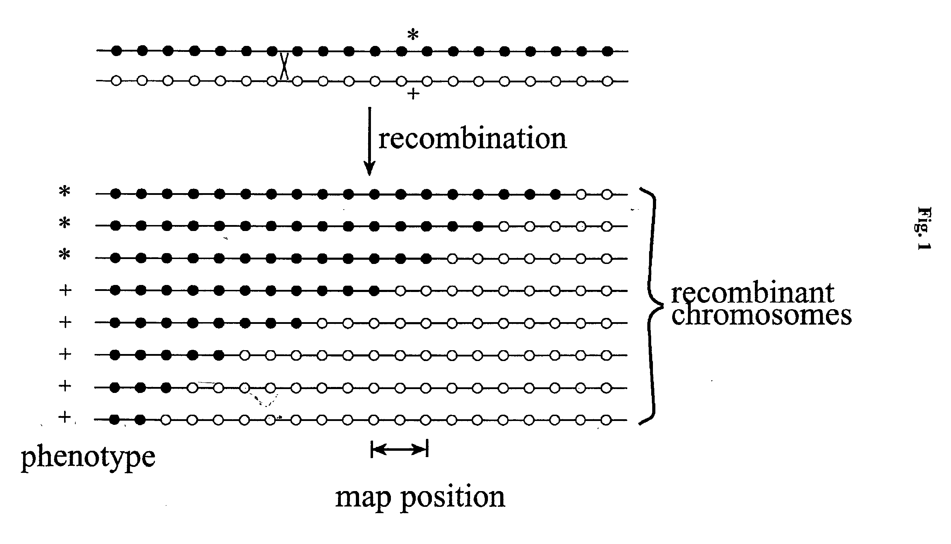

Fig. 1 shows the general principle of SNP mapping: The mutation to be mapped is indicated by the asterisk. The wild type allele at this locus on the chosen mapping strain is indicated by "+". These two chromosomes can be distinguished by a series of sequence polymorphisms (circles). Recombinant chromosomes are then derived by standard genetic crosses, yielding chromosomes consisting in part of each of the two parental chromosomes. The map position of the mutation is determined by scoring these recombinant chromosomes for the presence (*) or absence (+) of the mutation, and for the specific allele at selected polymorphic sites (black or white circle).

Several variations of the basic strategy may be made, e.g. as shown in the Example: the generation of recombinant chromosomes between a proximal and a distal P-element insertion on the same chromosome arm, as presented in Fig. 2; or the generation of recombinant chromosomes between the mutation of interest and a closely linked P-element insertion, as presented in Fig. 3.

In addition, recombinant chromosomes between two P element insertions may be generated that are both closely linked to and flank the mutation of interest. Each of these two P-elements may initially be placed either in trans or in cis to the mutation of interest.

The gene mapping methods developed in the experiments of the present invention are suitable to greatly accelerate the rate of positional cloning in *Drosophila*. The SNP map and PCR assays invention provide a means for fast, inexpensive, and high-resolution genetic mapping using existing laboratory strains of *Drosophila*. The inventors have demonstrated both the feasibility and accuracy of this method. In conjunction with the many methods currently being developed for high-throughput SNP genotyping (Kwok, 2001), the obtained SNP map now allows high-throughput high-resolution genetic mapping in *Drosophila.*

Positional cloning is no longer the rate-limiting step in the forward genetic analysis of complex biological phenomena.

Apart from its usefulness for mapping disease-related target genes, the gene mapping method of step A.3. can be generally used for mapping any Drosophila melanogaster gene.

The mapped Drosophila melanogaster gene can be cloned using any suitable cloning method, for example, hybridizing a probe with Drosophila melanogaster cDNA libraries (Rubin et al., 2000), rapid amplification of cDNA ends (RACE) (Frohman, PCR Methods App., (1994) *4:540-558*), and long-range PCR (Wilton *et al.*, Trends Genet (1996) 12:458).

Human or other mammalian homologues of this gene can then be identified by standard bioinformatics methods (homology searches) or by molecular approaches, e.g. by using probes from the Drosophila melanogaster DNA for cross-hybridizing with a mammalian, in particular human cDNA or genomic library.

As described in WO 01/51604, after amplification of a segment of the human orthologue using PCR or other suitable methods, that segment may be cloned and sequenced by standard techniques, and utilized to isolate a complete cDNA or genomic clone (Current Protocols in Molecular Biology, Vol. 1, Chap. 2.10, John Wiley & Sons, Publishers (1994); Sambrook *et al.*, Molecular Cloning, Cold Spring Harbor (1989); Frohman, see above). The gene product can be isolated using standard methods *(e.g.* ion exchange, affinity, and sizing column chromatography; centrifugation; differential solubility). The cDNA can be used for production of the deduced protein in recombinant form, according to standard methods. The amino acid sequence of the protein can be deduced from the nucleotide sequence. As a result, the protein can also be synthesized by standard chemical methods known in the art (Hunkapiller *et al.*, Nature (1984) 310:105-111).

The biological relevance of the identified gene and protein for the disease in question can be verified using either the Drosophila model, another animal model, or in various cellular assays. Examples for animal models are knock-out or conditional knock-out in mice. So-called "knock-down" approaches employ antisense oligonucleotide or RNAi (RNA interference). Also, the gene may be overexpressed. Small chemical compounds may also be used for validation of a target gene.

Depending upon the specific nature of the protein, cellular or biochemical assays can be used to identify small molecules that modulate, in particular inhibit, the function of this protein. The cDNA derived from the target gene, or the protein, are used in screening assays for modulators of its function.

**Brief description of the drawings**

Fig. 1: General principle of SNP mapping

Fig. 2: Genetic mapping with SNP markers

Fig. 3: High-resolution mapping

Fig. 4: Generation of recombinants using tandem P insertions

**Example**

The FRT strain and the EP strain that were used in the experiments were isogenized prior to construction of the SNP map. Canton S, Oregon R, and the strains with multiple recessive markers were obtained from the Bloomington Drosophila Stock Center. CyO and TM6B balancer chromosomes each carried a P{*yellow*+} insertion. The generation of these strains, and the isolation of the visual system connectivity mutants *2R-R971, 3L-H1135* and *3R-I256* are described in Newsome et al., 2000.

a) Choice of strategy for SNP identification

As outlined above when describing the general strategy, given the task of constructing a SNP map for two strains, A and B, and given a reference genome, G, one approach is to perform light shotgun sequencing of A, align these sequences to G, and determine the genotype of B for any polymorphic site thus identified. Alternatively, G might be used to design primers for the amplification and sequencing of selected loci from both A and B, which can then be directly compared. The cost per validated SNP for the shotgun (S) and directed (D) approaches can be estimated (see the general strategy above) by:

$$S = \frac{1}{f}\left(\frac{C_r}{(1-e^{-p_{AG}L})(1-r)} + 2(C_p+C_a)\right)$$

$$D = 2\left(\frac{C_p+2C_r}{s(1-e^{-2p_{AB}L})} + C_a\right)$$

where:

$C_r$=the cost of a sequencing reaction,

$C_p$=the cost of a primer for PCR and sequencing,

$C_a$=the cost of a genotyping assay (excluding primers),

$p_{XY}$=SNP frequency between strains X and Y,

L=read length,

r=repetitive fraction of the genome,

s=the success rate for genotyping assays using the original pair of primers, and

f=the probabilty of any A versus G polymorphism also being an A versus B polymorphism, given by the formula: $f=(1+(p_{AB}-p_{BG})/p_{AG})/2$.

The inventors have calculated D and S for a wide range of values for these parameters. Extreme values can be found under which, irrespective of polymorphism frequency ($p_{AB}$), either D is always less than S (e.g. a highly repetitive genome ($r\approx1$)), or S is always less than D (e.g. genotyping assays are difficult to design using the original primers ($s\approx0$), B is largely isogenic with G ($p_{BG}\approx0$), or primers and assays are much cheaper than sequencing reactions ($C_p,C_a\approx0$)). However, over a broad range of realistic values, there is a cutoff polymorphism frequency, above which D < S and below which S < D. Specifically, for the FRT and EP *Drosophila* strains, the inventors determined that the directed approach is favorable if one is interested in any SNP or only RFLP-SNPs, while the shotgun approach would be more suitable if one were interested only in larger ($\geq$7bp) InDels.

b) SNP identification

The inventors searched for sequence polymorphisms in each of the 10 autosomal contigs in Release 2 of the Drosophila genome with a length of at least 1 Mb (Adams *et al.*, 2000). Together, these contigs comprise 90 043 kb. Loci for amplification were selected from non-coding and non-repetitive regions evenly distributed across each contig. Noncoding regions were defined by the lack of any known or predicted open reading frame. Non-repetitive regions were defined by the presence of a single high-scoring BLAST hit on the Release 2 genome sequence.

PCR amplification was performed with genomic DNA prepared from FRT or EP homozygotes, primers (0.4µM), $MgCl_2$ (1.5mM), dNTPs (0.2mM each), and Taq DNA polymerase (TaKaRa, 1.25U). Cycle conditions were 94 °C for 5 minutes, followed by 35 cycles of 94 °C for 30 seconds, 62 °C for 30 seconds, and 72 °C for 2 minutes, and a final extension at 72 °C for 7 minutes. PCR was performed using either a GeneAmp 9700 (Perkin-Elmer) or Primus HT (MWG Biotech). PCR products were treated with ExoSAP-IT (US Biochemicals) to remove primers and excess nucleotides, and sequenced on an ABI377 or ABI3100 in two separate reactions, each using one of the PCR primers as a sequencing primer.

Individual sequence traces were called with PHRED 0.000925.c (Ewing et al., 1998a; Ewing et al., 1998b). Sequences were then preclustered into 1 Mb genomic intervals according to the initial PCR primer selection, and pair-wise alignments to the corresponding Release 2 genome sequence prepared for POLYBAYES using CrossMatch (Marth et al., 1999). Sequences were then multiply realigned using the POLYBAYES 3.0 (release 2001-03-27) anchored alignment algorithm[16]. Default parameters were used, with the exception that paralog filtering was unnecessary due to the preselection of unique regions, and therefore suppressed. The Release 2 genome sequence was assigned a PHRED score of 40. Polymorphisms reported by POLYBAYES were retained only if the PHRED quality score of each variant nucleotide exceeded 20, and the $P_{SNP}$ value assigned by POLYBAYES exceeded 0.5 (corresponding to a PHRED score of 33 in a pair-wise alignment). FRT and EP sequence traces were also aligned by the SeqMan program (DNAStar) and visually inspected. These alignments were used to make minor adjustments to the POLYBAYES alignment, and for the detection of most InDels larger than 8 nucleotides. SNPs were screened for the presence of recognition sites for a panel of 186 restriction endonucleases.

All PCR primers were designed using Primer3, which is available from Whitehead Institute/MIT Center for Genome Research (http://www-genome.wi.mit.edu/cgi-bin/primer/primer3_www.cgi). For SNP identification and PLP assays, primers were selected with melting temperatures of 67-73 °C, maximum $T_m$ difference 1 °C, length 20-30 nucleotides, 20-80% GC content, maximum self-complementarity 4.0, and maximum 3' self-complementarity 1.0. Primers for SNP identification were chosen to give a product of 1.0-1.3 kb.

c) PLP and FRLP assays

Genomic DNA was prepared either from 20-50 flies in 1ml reaction tubes, or from one or two flies in 96-well plates. For the former, DNA was resuspended in 50µl, of which 1-2µl used for PCR. For preparation in 96-well format, flies were manually crushed in 50µl of extraction buffer (10mM Tris-Cl, 1mM EDTA, 25mM NaCl, 200µg/ml Proteinase K) and incubated at 37 °C for 30 minutes. Proteinase K was then inactivated by incubation at 95 °C for 5 minutes, and 1µl used for PCR. PCR conditions were identical to those described above, except that extension times were reduced to 1 minute for PLP assays. For PLP assays, amplification products were separated on 2.5% agarose gels. For RFLP assays, 5μl PCR product was digested with the appropriate restriction enzyme in a 20μl reaction, and separated on a 1% agarose gel.

A set of 274 predicted RFLP-SNPs were selected for verification, based on the presence of a recognition site for a readily obtainable restriction endonuclease. 87% of these predicted RFLP-SNPs were experimentally confirmed. A negative result may be due to the presence of multiple recognition sites for the selected enzyme, similarities in the fragment sizes from the two strains, or a false prediction. It may be expected that, with optimal primer design, RFLP assays could be designed for more than 90% of the predicted RFLP-SNPs, allowing genotyping of 98% of those markers that contain at least one RFLP-SNP (89% of all markers).

For PLP assays, the product size was typically 130-230 bp (for InDels of up to 20bp) or 200-500 bp (for larger InDels). Primers used for SNP identification were also used for RFLP assays, although they are not necessarily the optimal choice for distinguishing the alternative fragment sizes. All oligonucleotides were synthesized by MWG Biotech.

d) High-resolution mapping of *2R-R971*

Recombinants were recovered between the *2R-R971* mutation and one of four selected P {*white*+} insertions in the 59DE region: *l(2)k07136, l(2)s4830, l(2)k06908*, or *l(2)k05606.* Recombinants were identified by screening for chromosomes that lacked the *white*+ marker but were viable in *trans* to *2R-J1609*, an independent mutation that has the same visual system connectivity defect, maps to 59DF, and fails to complement the lethality of *2R-R971. 71* recombinants were recovered from approximately 30,000 progeny.

References

Adams, M.D. *et al*. The genome sequence of *Drosophila melanogaster. Science* **287,** 2185-2195 (2000).

Begun, D.J. and Aquadro, C.F. Levels of naturally occurring DNA polymorphism correlate with recombination rates in *D. melanogaster. Nature* **356,** 519-520 (1992).

Benlali, A., Draskovic, I., Hazelett, D.J. & Treisman, J.E. *act up* controls actin polymerization to alter cell shape and restrict Hedgehog signaling in the *Drosophila* eye disc. *Cell* **101,** 271-281 (2000).

Brodsky, M. H., Nordstrom, W., Tsang, G., Kwan, E., Rubin, G. M., and Abrams, J. M. (2000). Drosophila p53 binds a damage response element at the reaper locus. Cell *101*, 103-13.

Chan, H. Y., and Bonini, N. M. (2000). Drosophila models of human neurodegenerative disease. Cell Death Differ 7, 1075-80.

Cho, R.J. *et al*. Genome-wide mapping with biallelic markers in *Arabidopsis thaliana. Nat. Genet.* **23,** 203-207 (1999).

Chou, T. B., Noll, E., and Perrimon, N. (1993). Autosomal P[ovoD1] dominant female-sterile insertions in Drosophila and their use in generating germ-line chimeras. Development *119*, 1359-69.

Collins, F.S., Guyer, M.S. & Charkravarti, A. Variations on a theme: cataloging human DNA sequence variation. *Science* **278,** 1580-1581 (1997).

Current Protocols in Molecular Biology, Vol. 1, Chap. 2.10, John Wiley & Sons, Pubhishers (1994)

Ewing, B., Hillier, L., Wendl, M.C. & Green, P. Base-calling of automated sequencer traces using phred. I. Accuracy assessment. *Genome Res.* **8**, 175-185 (1998a).

Ewing, B. & Green, P. Base-calling of automated sequencer traces using phred. II. Error probabilities. *Genome Res.* **8,** 186-194 (1998b).

Feany, M. B., and Bender, W. W. (2000). A Drosophila model of Parkinson's disease. Nature 404, 394-398.

Fernandez-Funez, P., Nino-Rosales, M. L., de Gouyon, B., She, W. C., Luchak, J. M., Martinez, P., Turiegano, E., Benito, J., Capovilla, M., Skinner, P. J., McCall, A., Canal, I., Orr, H. T., Zoghbi, H. Y., and Botas, J. (2000). Identification of genes that modify ataxin-1-induced neurodegeneration. Nature *408, 101-6.*

Frohman, PCR Methods App., (1994) *4:540-558*

Go and Artavanis-Tsakonas, Genetics (1998) 150: 211-220

Greenspan, R. Fly Pushing : The Theory and Practice of Drosophila Genetics, Cold Spring Harbor Laboratory Press, 1997

Hooper and Scott, Cell (1989) *59:751-765*

Hoskins, R.A. *et al*. Single Nucleotide Polymorphism Markers for Genetic Mapping in *Drosophila*

*melanogaster. Genome Res.* **11,** 1100-1113(2001).

Hunkapiller *et al.*, Nature (1984) 310:105-111

Jackson, G. R., Salecker, I., Dong, X., Yao, X., Arnheim, N., Faber, P. W., MacDonald, M. E., and Zipursky, S. L. (1998). Polyglutamine-expanded human huntingtin transgenes induce degeneration of Drosophila photoreceptor neurons. Neuron 21, 633-642.

Jin, S., Martinek, S., Joo, W. S., Wortman, J. R., Mirkovic, N., Sali, A., Yandell, M. D., Pavletich, N. P., Young, M. W., and Levine, A. J. (2000). Identification and characterization of a p53 homologue in Drosophila melanogaster. Proc Natl Acad Sci U S A 97, 7301-6.

Karim, F. D., Chang, H. C., Therrien, M., Wassarman, D. A., Laverty, T., and Rubin, G. M. (1996). A screen for genes that function downstream of Ras1 during Drosophila eye development. Genetics *143,* 315-29.

Karim, F. D., and Rubin, G. M. (1998). Ectopic expression of activated Ras1 induces hyperplastic growth and increased cell death in Drosophila imaginal tissues. Development *125*, 1-9.

Kimmerly, W. *et al.* A P1-based physical map of the *Drosophila* euchromatic genome. *Genome Res.* **6,** 414-430 (1996).

Kwok, P.-Y. Methods for Genotyping Single Nucleotide Polymorphisms. *Ann. Rev. Genomics Hum. Genet.* **2**, *235-258* (2001).

Lindblad-Toh, K. *et al.* Large-scale discovery and genotyping of single-nucleotide polymorphisms in the mouse. *Nat. Genet.* **24,** 381-386 (2000).

Liu, Y. and Montell, D.J. Identification of mutations that cause cell migration defects in mosaic clones. *Development* **126,** 1869-1878 (1999).

Marth, G.T. *et al.* A general approach to single-nucleotide polymorphism discovery. *Nat. Genet.* **23,** 452-456 (1999).

Min, K. T., and Benzer, S. (1999). Preventing neurodegeneration in the Drosophila mutant bubblegum. Science *284*, 1985-8.

Moberg, K. H., Bell, D. W., Wahrer, D. C., Haber, D. A., and Hariharan, I. K. (2001). Archipelago regulates Cyclin E levels in Drosophila and is mutated in human cancer cell lines. Nature *413,* 311-6.

Neuman-Silberberg, F. S., Schejter, E., Hoffmann, F. M., and Shib, B. Z. (1984). The Drosophila ras oncogenes: structure and nucleotide sequence. Cell *37,* 1027-33.

Newsome, T. P., Asling, B., and Dickson, B. J. (2000). Analysis of Drosophila photoreceptor axon guidance in eye-specific mosaics. Development *127*, 851-860.

Ollmann, M., Young, L. M., Di Como, C. J., Karim, F., Belvin, M., Robertson, S., Whittaker, K., Demsky, M., Fisher, W. W., Buchman, A., Duyk, G., Friedman, L., Prives, C., and Kopczynski, C. (2000). Drosophila p53 is a structural and functional homolog of the tumor suppressor p53. Cell *101*, 91-101.

Pichaud, F. and Desplan, C. A new visualization approach for identifying mutations that affect differentiation and organization of the *Drosophila* ommatidia. *Development* **128,** 815-826 (2001).

Raftery *et al.*, Genetics 139:241-254 (1995)

Rorth, P. A modular misexpression screen in Drosophila detecting tissue-specific phenotypes. Proc Natl Acad Sci U S A 93, 12418-22 (1996).

Rørth, P. *et al.* Systematic gain-of-function genetics in *Drosophila. Development* **125,** 1049-1057 (1998).

Ruan, W., Pang, P. & Rao, Y. The SH2/SH3 adaptor protein dock interacts with the Ste20-like kinase misshapen in controlling growth cone motility. *Neuron* **24,** 595-605 (1999).

Rubin, G. M., Hong, L., Brokstein, P., Evans-Holm, M., Frise, E., Stapleton, M., and Harvey, D. A. (2000a). A Drosophila complementary DNA resource. Science *287,* 2222-4.

Rubin, G. M., Yandell, M. D., Wortman, J. R., Gabor Miklos, G. L., Nelson, C. R., Hariharan, I. K., Fortini, M. E., Li, P. W., Apweiler, R., Fleischmann, W., Cherry, J. M., Henikoff, S., Skupski, M. P., Misra, S., Ashburner, M., Birney, E., Boguski, M. S., Brody, T., Brokstein, P., Celniker, S. E., Chervitz, S. A., Coates, D., Cravchik, A., Gabrielian, A., Galle, R. F., Gelbart, W. M., George, R. A., Goldstein, L. S., Gong, F., Guan, P., Harris, N. L., Hay, B. A., Hoskins, R. A., Li, J., Li, Z., Hynes, R. O., Jones, S. J., Kuehl, P. M., Lemaitre, B., Littleton, J. T., Morrison, D. K., Mungall, C., O'Farrell, P. H., Pickeral, O. K., Shue, C., Vosshall, L. B., Zhang, J., Zhao, Q., Zheng, X. H., Zhong, F., Zhong, W., Gibbs, R., Venter, J. C., Adams, M. D., and Lewis, S. (2000b). Comparative genomics of the eukaryotes. Science 287, 2204-2215.

Sambrook *et al.*, Molecular Cloning, Cold Spring Harbor (1989)

Smith, D., Wohlgemuth, J., Calvi, B. R., Franklin, I., and Gelbart, W. M. (1993). hobo enhancer trapping mutagenesis in Drosophila reveals an insertion specificity different from P elements. Genetics *135,* 1063-76.

Spradling, A. C., Stern, D. M., Kiss, I., Roote, J., Laverty, T., and Rubin, G. M. (1995). Gene disruptions using P transposable elements: an integral component of the Drosophila genome project. Proc Natl Acad Sci U S A 92, 10824-30.

Spradling, A. C., Stern, D., Beaton, A., Rhem, E. J., Laverty, T., Mozden, N., Misra, S., and Rubin, G. M. (1999). The Berkeley Drosophila Genome Project gene disruption project: Single P-element insertions mutating 25% of vital Drosophila genes. Genetics *153,* 135-77.

Tapon, N., Ito, N., Dickson, B. J., Treisman, J. E., and Hariharan, I. K. (2001). The Drosophila tuberous sclerosis complex gene homologs restrict cell growth and cell proliferation. Cell *105,* 345-55.

Teeter, K. *et al*. Haplotype dimorphism in a SNP collection from *Drosophila melanogaster. J. Exp. Zool.* **288,** 63-75 (2000).

Warrick, J. M., Paulson, H. L., Gray-Board, G. L., Bui, Q. T., Fischbeck, K. H., Pittman, R. N., and Bonini, N. M. (1998). Expanded polyglutamine protein forms nuclear inclusions and causes neural degeneration in Drosophila. Cell 93, 939-49.

Wicks, S.R., Yeh, R.T., Gish, W.R., Waterston, R.H. & Plasterk, R.H. Rapid gene mapping in *Caenorhabditis elegans* using a high density polymorphism map. *Nat. Genet.* **28,** 160-164 (2001).

Wilton *et al.*, Trends Genet (1996) 12:458.

Winzeler, E.A. *et al*. Direct allelic variation scanning of the yeast genome. *Science* **281,** 1194-1197 (1998).

Wittmann, C. W., Wszolek, M. F., Shulman, J. M., Salvaterra, P. M., Lewis, J., Hutton, M., and Feany, M. B. (2001). Tauopathy in Drosophila: neurodegeneration without neurofibrillary tangles. Science 293, 711-4.

Xu, T., and Rubin, G. M. (1993). Analysis of genetic mosaics in developing and adult Drosophila tissues. Development *117*, 1223-37.

Xu, T., Wang, W., Zhang, S., Stewart, R. A., and Yu, W. (1995). Identifying tumor suppressors in genetic mosaics: the Drosophila lats gene encodes a putative protein kinase. Development *121,* 1053-1063.

**Claims**

1. A method for producing a human cDNA molecule derived from a gene that is causally involved in a human disease by

   A. identifying a Drosophila melanogaster gene that is involved in a molecular process or pathway related to a disease of interest by

      1. obtaining genetically altered flies that exhibit a disease-related phenotype by

         a) expressing in flies a human gene known to be implicated in the disease, or its Drosophila homologue, is in its normal or mutated form such that expression of the gene results in a gene-specific phenotype; or

         b) randomly mutating flies and identifying flies that exhibit a disease-related phenotype;

      2. mutagenizing animals obtained in 1.a) and recovering mutations that modify said phenotype of 1.a); or recovering animals that result in phenotype 1.b);

      3. mapping the mutated Drosophila gene, and

      4. cloning the Drosophila gene;

   B. identifying the orthologue human gene and cloning and amplifying the human cDNA,

   **characterized in that** step A.3 comprises the steps of

      (a) setting up a genetic cross between the Drosophila strain containing the mutation recovered in step 2., and another strain that is sufficiently divergent at the DNA sequence level, and that contains one or more P element insertions that allow recombination events within a defined genomic interval to be identified;

      (b) recovering a series of recombinant progeny from this cross, each of which contains a genome that is a mixture of the two parental strains;

(c) identifying a set of sequence polymorphisms that discriminates between the two parental strains;

(d) determining the phenotype of some or all of the recombinant progeny;

(e) assaying for some or all of the sequence polymorphisms identified in (c) in some or all of the recombinant progeny.

2. The method of claim 1, wherein in step A.3.a) two strains are used each of which carries a P element located within 1 Mb of the centromere or the telomere, thereby generating a set of recombinants spanning an entire chromsome arm.

3. The method of claim 2, whererein in step A.3.a) the strain carrying the mutation of interest is a FRT strain and the other strain is an EP strain carrying an EP element distal to the mutation.

4. The method of claim 3, wherein the EP strain carries an EP element that lies distal to but near the mutation of interest and that has been identied from a series of EP elements across the chromosome arm by phenotypic analysis of recombinants between the mutated strain and the EP strain.

5. The method of claim 1, wherein in step A.3.b) the recombinants are reocovered due to the expression of an FLP-recombinase that is under the control of an eye-specific enhancer so that recombinants are identified by their mosaicism for an eye-colour marker.

6. The method of claim 1, wherein in step A.3.c) the polymorphisms are identified by selecting regions from a set of paired Drosophila melanogaster strains for each chromosome arm, amplifying the selected regions, sequencing the amplified DNA from each pair of homozygotes and determining polymorphisms between the two strains.

7. The method of claim 6, wherein the Drosophila strains are a FRT and an EP strain.

8. The method of claim 1 or 7, wherein the polymorphisms of step A.3.c) and A.3.d) are selected from the set listed in Tables A - D.

9. The method of claim 1, wherein in step A.3.e) the recombinant progeny is assayed for polymorphism (s) by PCR product length polymorphisms (PLP).

10. The method of claim 9, wherein the recombinant progeny is assayed for polymorphism(s) listed in Tables A - D by the corresponding PLP assay(s) of Table E.

11. The method of claim 1, wherein in step A.3.e) the recombinant progeny is assayed for polymorphism (s) by restriction fragment length polymorphisms (RFLP).

12. The method of claim 11, wherein the recombinant progeny is assayed for polymorphism(s) listed in Tables A - D by the corresponding RFPL assay(s) of Table F.

13. The method of claim 1, wherein the phenotype of step A.1. is associated with lethality and in step A. 3.d) recombinant progeny is recovered by selecting against the mutation-associated lethality.

14. The method of claim 1, wherein the recombinant progeny in step A.3.d) are recovered by selecting for recombination between two transposon insertions separated by less than 4 Mb, in either the cis or the trans configuration.

15. A method for mapping a mutated gene in Drosophila melanogaster, comprising the steps of

(a) setting up a genetic cross between the Drosophila strain containing the mutation and another strain that is sufficiently divergent at the DNA sequence level, and that contains one or more P element insertions that allow recombination events within a defined genomic interval to be identified;

(b) recovering a series of recombinant progeny from this cross, each of which contains a genome that is a mixture of the two parental strains;

(c) identifying a set of sequence polymorphisms that discriminates between the two parental strains;

(d) determining the phenotype of some or all of the recombinant progeny;

(e) assaying for some or all of the sequence polymorphisms identified in (c) in some or all of the recombinant progeny.

16. The method of claim 15, wherein in step a) two strains are used each of which carries a P element located within 1 Mb of the centromere or the telomere, thereby generating a set of recombinants spanning an entire chromsome arm.

**17.** The method of claim 16, whererein in step a) the strain carrying the mutation is a FRT strain and the other strain is an EP strain element distal to the mutation.

**18.** The method of claim 17, wherein the EP strain carries an EP element that lies distal to but near the mutation of interest and that has been identied from a series of EP elements across the chromosome arm by phenotypic analysis of recombinants between the mutated strain and the EP strain.

**19.** The method of claim 15, wherein in step b) the recombinants are reocovered due to the expression of an FLP-recombinase that is under the control of an eye-specific enhancer so that recombinants are identified by their mosaicism for an eye-colour marker.

**20.** The method of claim 15, wherein in step c) the polymorphisms are identified by selecting regions from a set of paired Drosophila melanogaster strains for each chromosome arm, amplifying the selected regions, sequencing the amplified DNA from each pair of homozygotes and determining polymorphisms between the two strains.

**21.** The method of claim 20, wherein the Drosophila strains are a FRT and an EP strain.

**22.** The method of claim 15 or 20, wherein the polymorphisms of step c) and d) are selected from the set listed in Tables A - D.

**23.** The method of claim 15, wherein in step e) the recombinant progeny is assayed for polymorphism(s) by PCR product length polymorphisms (PLP).

**24.** The method of claim 15, wherein in step e) the recombinant progeny is assayed for polymorphism(s) by restriction fragment length polymorphisms (RFLP).

**25.** The method of claim 23, wherein the recombinant progeny is assayed for polymorphism(s) listed in Tables A - D by the corresponding PLP assay(s) of Table E.

**26.** The method of claim 24, wherein the recombinant progeny is assayed for polymorphism(s) listed in Tables A - D by the corresponding RFPL assay(s) of Table F.

**27.** The method of claim 15, wherein the mutation is associated with lethality and in step d) recombinant progeny is recovered by selecting against the mutation-associated lethality.

**28.** The method of claim 15, wherein the recombinant progeny are recovered by selecting for recombination between two transposon insertions separated by less than 4 Mb, in either the cis or the trans configuration.

**29.** The use of the set of polymorphisms listed in Tables A - D , or a subset thereof, for mapping genes in Drosophila melanogaster.

**30.** The use of a PCR product length polymorphisms (PLP) assay of Table E for for determining a polymorphism in Drosophila melanogaster.

**31.** The use of a restriction fragment length polymorphism (RFLP) assay of Table F for determining a polymorphism in Drosophila melanogaster.

Fig. 1

**Fig. 2 a,b**

## Fig 2c,d

*misshapen*

169 kb

307 kb

Fig. 3

# Fig. 4

**European Patent Office**

## PARTIAL EUROPEAN SEARCH REPORT

**Application Number**

which under Rule 45 of the European Patent Convention shall be considered, for the purposes of subsequent proceedings, as the European search report

EP 01 12 7195

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X,D | HOSKINS ROGER A ET AL: "Single nucleotide polymorphism markers for genetic mapping in Drosophila melanogaster." GENOME RESEARCH, vol. 11, no. 6, June 2001 (2001-06), pages 1100-1113, XP002201468 ISSN: 1088-9051 * whole document * | 1,2,6,9, 11, 13-16, 20,23, 24,27,28 | C12Q1/68 |
| Y,D | TEETER KATHERINE ET AL: "Haplotype dimorphism in a SNP collection from Drosophila melanogaster." JOURNAL OF EXPERIMENTAL ZOOLOGY, vol. 288, no. 1, 15 April 2000 (2000-04-15), pages 63-75, XP002201469 ISSN: 0022-104X * whole document * | 15-21, 23,24, 27,28 | |
| | -/-- | | |

**TECHNICAL FIELDS SEARCHED (Int.Cl.7)**

C12Q

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 11 June 2002 | Aguilera, M |

| European Patent Office | INCOMPLETE SEARCH SHEET C | Application Number EP 01 12 7195 |
|---|---|---|

Claim(s) searched completely:
    1-7,9,11,13-21,23,24,27,28

Claim(s) not searched:
    8,10,12,22,25,26,29-31

Reason for the limitation of the search:

Present claims 8,10,12,22,25,26 and 29-31 relate to an extremely large number of possible methods; virtually, any method for mapping a Drosophila modifier locus using any of the 8615 polymorphisms selected from Tables A-F, or any combination of them, as markers for the genotyping of recombinants. In fact, the claims contain so many possible combinations that a lack of clarity and conciseness within the meaning of Article 84 EPC arises to such an extent as to render a meaningful search over the whole of the claimed scope impossible.

Consequently, the search has been carried out only for those claims which do appear to be clear and concise, namely claims 1-7, 9, 11, 13-21, 23, 24, 27 and 28.

**European Patent Office**

## PARTIAL EUROPEAN SEARCH REPORT

Application Number

EP 01 12 7195

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| Y | ZIMMERMAN ERIKA ET AL: "Quantitative trait loci affecting components of wing shape in Drosophila melanogaster." GENETICS, vol. 155, no. 2, June 2000 (2000-06), pages 671-683, XP002201470 ISSN: 0016-6731 * page 671 - page 672; table 1 * | 15-21, 23,24, 27,28 | |
| A,D | WO 01 51604 A (DUYK GEOFFREY ;FRIEDMAN LORI (US); KARIM FELIX D (US); EXELIXIS IN) 19 July 2001 (2001-07-19)<br><br>* whole document * | 1-7,9, 11, 13-21, 23,24, 27,28 | |
| A | WANG D G ET AL: "Large-scale identification, mapping, and genotyping of single-nucleotide polymorphisms in the human genome" SCIENCE, AMERICAN ASSOCIATION FOR THE ADVANCEMENT OF SCIENCE,, US, vol. 280, 1998, pages 1077-1082, XP002089398 ISSN: 0036-8075 * whole document * | 1-7,9, 11, 13-21, 23,24, 27,28 | TECHNICAL FIELDS SEARCHED (Int.Cl.7) |
| A,D | XU TIAN ET AL: "Analysis of genetic mosaics in developing and adult Drosophila tissues." DEVELOPMENT (CAMBRIDGE), vol. 117, no. 4, 1993, pages 1223-1237, XP002201471 ISSN: 0950-1991 * whole document * | 1-7,9, 11, 13-21, 23,24, 27,28 | |

-/--

**European Patent Office**

## PARTIAL EUROPEAN SEARCH REPORT

Application Number

EP 01 12 7195

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| A | KOCH ROMKE ET AL: "Single nucleotide polymorphisms in wild isolates of Caenorhabditis elegans." GENOME RESEARCH, vol. 10, no. 11, November 2000 (2000-11), pages 1690-1696, XP001077876 ISSN: 1088-9051 * whole document * | 1-7,9, 11, 13-21, 23,24, 27,28 | |
| T | BERGER JURG ET AL: "Genetic mapping with SNP markers in Drosophila." NATURE GENETICS, vol. 29, no. 4, December 2001 (2001-12), pages 475-481, XP002201472 ISSN: 1061-4036 * whole document * | 1-7,9, 11, 13-21, 23,24, 27,28 | **TECHNICAL FIELDS SEARCHED** (Int.Cl.7) |

EPO FORM 1503 03.82 (P04C10)

## ANNEX TO THE EUROPEAN SEARCH REPORT
## ON EUROPEAN PATENT APPLICATION NO.

EP 01 12 7195

This annex lists the patent family members relating to the patent documents cited in the above-mentioned European search report.
The members are as contained in the European Patent Office EDP file on
The European Patent Office is in no way liable for these particulars which are merely given for the purpose of information.

11-06-2002

| Patent document cited in search report | | Publication date | Patent family member(s) | | Publication date |
|---|---|---|---|---|---|
| WO 0151604 | A | 19-07-2001 | AU | 3088801 A | 24-07-2001 |
| | | | WO | 0151604 A2 | 19-07-2001 |

EPO FORM P0459

For more details about this annex : see Official Journal of the European Patent Office, No. 12/82